# EUROPÄISCHE PATENTSCHRIFT

(11) **EP 1 723 172 B1**
(45) Veröffentlichungstag und Bekanntmachung des Hinweises auf die Patenterteilung: **08.08.2007**
(21) Anmeldenummer: 05707735.6
(22) Anmeldetag: 10.03.2005
(51) Int. Cl.: C07K 14/505, A61K 47/18

(54) **ERYTHROPOIETIN-FLÜSSIGFORMULIERUNG**
ERYTHROPOIETIN LIQUID FORMULATION
FORMULATION LIQUIDE D'ERYTHROPOIETINE

(30) Priorität: 10.03.2004 DE 102004011663
(43) Veröffentlichungstag der Anmeldung: 22.11.2006
(60) Teilanmeldung: 07106020.6
(73) Patentinhaber: Bioceuticals Arzneimittel AG, 61118 Bad Vilbel (DE)
(72) Erfinder: SCHUMANN, Christof, 35767 Breitscheid-Erdbach (DE); HESSE, Jan-Ole, 61231 Bad Nauheim (DE)
(74) Vertreter: Neuefeind, Regina
(86) Internationale Anmeldenummer: PCT/EP2005/002551
(87) Internationale Veröffentlichungsnummer: WO 2005/087804

(56) Entgegenhaltungen:
- EP-A- 0 178 665
- EP-A- 0 306 824
- EP-A- 0 909 564
- WO-A-00/61169
- US-A1- 2003 162 711

## Beschreibung

Die vorliegende Erfindung betrifft lagerstabile Erythropoietin-Flüssigformulierungen und Verfahren zu deren Herstellung. Insbesondere betrifft die Erfindung Erythropoietin-Flüssigformulierungen, die mindestens vier Aminosäuren ausgewählt aus der Gruppe bestehend aus Leucin, Isoleucin, Threonin, Glutaminsäure, Asparaginsäure und Phenylalanin enthalten und in denen auf die Zugabe von Konservierungsmitteln, Harnstoff oder Humanserumalbumin verzichtet werden kann.

Erythropoietin, kurz EPO genannt, ist ein Glykoprotein, das die Bildung von Erythrocyten im Knochenmark anregt. EPO wird hauptsächlich in den Nieren gebildet und gelangt von dort aus über den Blutkreislauf zu seinem Zielort. Bei Niereninsuffizienz produzieren die geschädigten Nieren zu wenig oder überhaupt kein EPO, was zur Folge hat, dass aus den Stammzellen des Knochenmarks zu wenig Erythrocyten hervorgehen. Diese renale Anämie kann durch Verabreichung von EPO in physiologischen Mengen, die die Bildung von Erythrocyten im Knochenmark stimulieren, behandelt werden. Das zur Verabreichung verwendete EPO kann entweder aus Humanurin gewonnen oder durch gentechnologische Methoden erzeugt werden. Da EPO im menschlichen Körper nur in geringen Spuren enthalten ist, ist die Isolierung von EPO aus der natürlichen Quelle für therapeutische Anwendungen praktisch unmöglich. Daher bieten gentechnische Methoden die einzige wirtschaftliche Möglichkeit, diesen Stoff in größeren Mengen zu produzieren.

Die rekombinante Herstellung von Erythropoietin ist seit der Identifizierung des humanen Erythropoietin-Gens im Jahr 1984 möglich. Seit Anfang der 90er Jahre sind verschiedene Arzneimittel entwickelt worden, die humanes Erythropoietin enthalten, das auf gentechnologischem Weg in eukaryontischen Zellen, vor allem in CHO-Zellen (Chinese Hamster Ovary) produziert wurde. Die Herstellung von rekombinantem humanen Erythropoietin ist beispielsweise beschrieben in EP-A-0 148 605 und EP-A-205 564.

EPO wird üblicherweise in flüssiger Form formuliert und wird als solche intravenös oder subkutan injiziert. Allerdings ergeben sich bei der Flüssigformulierung von EPO Probleme hinsichtlich der Stabilität. Diese sind möglicherweise auf eine Zerstörung der Erythropoietin-Moleküle durch katalytische Effekte der Oberfläche der zur Aufbewahrung dienenden Behältnisse zurückzuführen. Des Weiteren wird vermutet, dass eine Adsorption der EPO-Moleküle an die Gefäßwand eintritt und es hierdurch zu einem Verlust an Protein und Aktivität kommt. Daher wurden in der Vergangenheit verschiedene Ansätze verfolgt, Flüssigformulierungen von EPO durch Zusatz verschiedener Stoffe zu stabilisieren. Zu diesen stabilisierenden Verbindungen gehören zum Beispiel polymere Substanzen wie Polyethylenglykol, Dextrane und Gelatine, sowie verschiedene Zucker und Zuckeralkohole, anorganische Salze und Thiolverbindungen. Häufig werden auch Serumproteine wie Humanserumalbumin (HSA) zugesetzt. Die Verwendung dieser Serumproteine ist allerdings aus allergener und immunologischer Sicht nicht wünschenswert.

In EP-A-0 306 824 wird die Verwendung verschiedener Aminosäuren als Stabilisatoren in EPO-Formulierungen beschrieben, wobei die Aminosäuren zusammen mit Harnstoff zur Stabilisierung des EPO eingesetzt werden. Zudem erfolgt die Lagerung der in EP-A-0 306 824 beschriebenen Formulierungen nicht in flüssiger Form, sondern als Lyophilisat, das unmittelbar vor der Anwendung mit Wasser rekonstituiert wird.

In EP-A-0 607 156 werden EPO-Formulierungen beschrieben, die dank der Gegenwart eines Konservierungsmittel stabil und haltbar sind.

Aufgabe der vorliegenden Erfindung ist es, eine lagerstabile EPO-Zubereitung herzustellen, die in flüssiger Form über längere Zeit gelagert werden kann und die ohne Zusatzstoffe wie HSA, Harnstoff oder Konservierungsmittel auskommt. Auf diese Weise soll eine stabile EPO-Flüssigformulierung bereitgestellt werden, die möglichst jedes Risiko für die Verträglichkeit der Formulierung vermeidet.

Diese Aufgabe wird erfindungsgemäß gelöst durch den Gegenstand des Anspruchs 1. Bevorzugte Ausführungsformen sind in den Unteransprüchen definiert.

Es wurde gefunden, dass EPO-Zusammensetzungen, die eine bestimmte Kombination von Aminosäuren enthalten, auch bei Abwesenheit von HSA, Harnstoff oder polymeren Stabilisierungsmitteln in flüssiger Form über längere Zeit stabil gelagert werden können, ohne dass es zu signifikanten Stabilitätsverlusten kommt.

Die vorliegende Erfindung betrifft somit eine lagerstabile Erythropoietin-Flüssigformulierung, die
(i) mindestens vier Aminosäuren, ausgewählt aus der Gruppe bestehend aus Leucin, Isoleucin, Threonin, Glutaminsäure, Asparaginsäure und Phenylalanin enthält,
(ii) frei von Konservierungsmitteln, Harnstoff und Serumproteinen ist, und
(iii) nicht aus einem Lyophilisat rekonstituiert wurde.

In einer besonders bevorzugten Ausführungsform ist zusätzlich die Aminosäure Glycin enthalten.

Die erfindungsgemäße Flüssigformulierung bietet nicht nur die Vorteile, dass sie auf potentiell immunogene Verbindungen verzichtet und insgesamt eine möglichst geringe Anzahl von verschiedenen Verbindungsklassen enthält, sondern sie erfordert auch in keiner Phase der Herstellung eine Lyophilisierung. Hierdurch werden zum einen die mit einer Lyophilisierung verbundenen Kosten eingespart, zum anderen werden die Risiken mechanischer Probleme, z.B. wenn sich das Lyophilisat nicht in vollständiger oder ausreichender Weise rekonstituieren lässt, vermieden.

Unter dem Begriff "lagerstabil" wird im Rahmen der vorliegenden Erfindung verstanden, dass der Gehalt an aktiven EPO-Molekülen nach dreimonatiger Lagerung der EPO-Flüssigformulierung bei 25°C immer noch 80% oder mehr der Ausgangskonzentration beträgt. Vorzugsweise beträgt der Restgehalt an EPO-Aktivität nach dreimonatiger Lagerung bei 25°C noch mindestens 85%, bevorzugter mindestens 90% und am meisten bevorzugt mindestens 95% der Ausgangsaktivität. Die Aktivität des Erythropoietins kann mittels üblicher Aktivitätstests, wie sie bereits für EPO im Stand der Technik beschrieben sind, bestimmt werden, siehe ergänzend auch Europäische Pharmacopoeia, 01/2002:1316 (4. Auflage).

Unter dem Begriff "Flüssigformulierung" wird im Rahmen der vorliegenden Erfindung verstanden, dass die Formulierung von Erythropoietin zusammen mit den anderen in der Formulierung enthaltenen Stoffen in keiner Phase des Herstellungsprozesses, also weder vor noch während oder nach dem Mischen der Stoffe, lyophilisiert wird und die Formulierung für die intravenöse oder subkutane Applikation als Injektions- oder Infusionslösung bestimmt ist.

Die erfindungsgemäße Formulierung enthält keine Aminosäuren, bei denen es sich nicht um Leucin, Isoleucin, Threonin, Glutaminsäure, Asparaginsäure, Phenylalanin oder Glycin handelt. Bevorzugt enthält die Formulierung keine anderen Aminosäuren als Leucin, Isoleucin, Threonin, Glutaminsäure, Phenylalanin und Glycin. Insbesondere enthält die EPO-Formulierung gemäß der vorliegenden Erfindung keine basischen Aminosäuren. Ebenfalls enthält die Formulierung keine Aminoessigsäure. Die zwingende Abwesenheit von Serumproteinen oder anderen stabilisierenden Proteinen, von Harnstoff und Konservierungsmitteln in der erfindungsgemäßen Formulierung wurde bereits oben angesprochen.

Die Konzentration von Isoleucin und Leucin, soweit diese Aminosäuren in der Formulierung enthalten sind, beträgt jeweils 0,25 bis 1,5 g/l, bevorzugt 0,5 bis 1,25 g/l und besonders bevorzugt 1,0 g/l.

Die Konzentration von Glutaminsäure und Asparaginsäure, soweit diese Aminosäuren in der Formulierung enthalten sind, beträgt 0,1 bis 0,5 g/l, bevorzugt 0,2 bis 0,4 g/l und besonders bevorzugt 0,25 g/1.

Die Konzentration von Threonin, soweit diese Aminosäure in der Formulierung enthalten ist, beträgt 0,1 bis 0,5 g/l, bevorzugt 0,2 bis 0,4 g/l, besonders bevorzugt 0,25 g/l.

Die Konzentration von Phenylalanin, soweit diese Aminosäure in der Formulierung enthalten ist, beträgt 0,2 bis 1,0 g/l, bevorzugt 0,3 bis 0,8 g/l und besonders bevorzugt 0,5 g/l.

Die Konzentration von Glycin in der erfindungsgemäßen Formulierung beträgt, soweit diese Aminosäure in der Formulierung enthalten ist, 2,0 bis 10,0 g/l, bevorzugt 5,0 bis 8,0 g/l und besonders bevorzugt 7,5 g/l.

Das Erythropoietin, das sich zur Verwendung in den erfindungsgemäßen Formulierungen eignet, ist rekombinantes Erythropoietin, hergestellt in eukaryontischen Zellen. Bevorzugt wird das rekombinante Erythropoietin in Säugerzellen, besonders bevorzugt in CHO-Zellen hergestellt, wie z.B. beschrieben in EP-A-0 205 564 und EP-A-0 148 605. Nach der Fermentation, die nach herkömmlichen Protokollen erfolgt, findet eine Aufreinigung des rekombinanten Erythropoietins mittels chromatographischer Methoden statt. Sowohl die Fermentierung als auch die Reinigung des Proteins sind ebenfalls im Stand der Technik beschrieben, z.B. in EP-A-0 830 376. Die Reinigung von Erythropoietin ist auch Gegenstand von EP-A-0 228 452, EP-A-0 428 267 und WO-A-03/045996. Aber auch andere übliche Reinigungstechniken und Abfolgen von Reinigungsschritten können angewandt werden.

Unter "Erythropoietin" wird im Rahmen der vorliegenden Erfindung jedes Protein verstanden, das in der Lage ist, die Erythrocyten-Bildung im Knochenmark zu stimulieren und gemäß dem in der Europäischen Pharmacopoeia (Ph. Eur.; O1/2002:1316) beschriebenen Assay eindeutig als Erythropoietin identifiziert werden kann (Bestimmung der Aktivität in polycythämischen oder normocythamischen Mäusen). Bei dem Erythropoietin kann es sich um das wildtypische humane Eythropoietin oder um eine Variante davon mit einem oder mehreren Aminosäureaustauschen, -deletionen oder -additionen handeln. Ebenso kann es sich bei dem in der erfindungsgemäßen Formulierung enthaltenen Erythropoietin um ein Konjugat handeln, in dem das Protein in konjugierter Form z.B. mit Polymeren wie etwa Polyalkylenglykolen vorliegt, sog. PEGyliertes Erythropoietin.

Die erfindungsgemäße Formulierung enthält in einer bevorzugten Ausführungsform weder Zucker noch Zuckeralkohole zur Stabilisierung. Es ist ebenfalls bevorzugt, dass die Formulierung keine polymeren Verbindungen als Stabilisierungsmittel enthält. Die Formulierung ist zudem frei von Konservierungsmitteln, wobei hierunter Substanzen verstanden werden, die üblicherweise als Konservierungsmittel zur Erhöhung der Haltbarkeit eingesetzt werden und in den dabei üblichen Konzentrationen bakterizid wirken. Insbesondere enthält die Formulierung keine Konservierungsmittel wie Chlorethon, Benzylalkohol, pChlor-m-Kresol und Pyrokohlensäure-Dialkylester und Benzallconiumchlorid.

Der pH-Wert der Formulierung liegt nicht wesentlich über dem pH-Wert des Blutes, also möglichst nicht über 7,4. Der pH-Wert der Formulierung beträgt in der Regel zwischen 6,0 und 7,4, bevorzugt zwischen 6,5 und 7,4 und besonders bevorzugt zwischen 7,0 und 7,4. Der pH-Wert wird, falls eine Einstellung in den angestrebten pH-Bereich erforderlich ist, mit geeigneten Lösungen eingestellt, wenn eine Erniedrigung des pH-Werts angezeigt ist, mit sauren Lösungen, wenn eine Erhöhung erzielt werden soll, mit basischen Lösungen. Bevorzugt wird der pH-Wert mit HCI bzw. NaOH eingestellt.

Als Lösungsmittel wird bevorzugt reines Wasser für Injekionszwecke verwendet. Aber auch andere für pharmazeutische Zubereitungen geeignete und übliche Lösungsmittel können eingesetzt werden.

Bei dem eingesetzten Puffer kann es sich um jeden physiologisch verträglichen Puffer handeln, der es ermöglicht, in den für Injektions- bzw. Infusionslösungen erforderlichen Konzentrationen in dem oben angegebenen pH-Bereich zu puffern. Geeignete Puffer sind z.B. Phosphat, Citrat, Carbonat und HEPES. Die Verwendung eines Phosphatpuffers ist bevorzugt. Die Pufferverbindung bzw. -verbindungen werden in einer Konzentration von etwa 20 bis 100 mM, bevorzugt 30 bis 80 mM und besonders bevorzugt 40 bis 60 mM eingesetzt.

Als weitere Komponente enthält die erfindungsgemäße Formulierung in einer bevorzugten Ausführungsform ein oberflächenaktives Mittel. Die Verwendung eines Detergens soll die Adsorption von EPO an die Gefäßwände verringern. Hierzu reichen in der Regel geringe Mengen eines Detergens aus. Grundsätzlich kann jedes physiologisch verträgliche Detergens im Rahmen der vorliegenden Erfindung eingesetzt werden. Besonders geeignet sind Polyoxyethylensorbitanalkylester oder Sorbitantrioleat, wobei Polyoxyethylensorbitanalkylester besonders bevorzugt sind. Bei dem Polyoxyethylensorbitanalkylester handelt es sich bevorzugt um Polysorbat 20 und/oder Poylsorbat 80, wobei Polysorbat 20 am meisten bevorzugt ist. Die Konzentration des Detergens beträgt zwischen 0,01 bis 1,0 g/l, bevorzugt zwischen 0,05 und 0,2 g/l und am meisten bevorzugt 0,1 g/l.

Weiterhin enthält die Formulierung in bevorzugten Ausführungsformen geringe Mengen eines physiologisch verträglichen Komplexbildners. Bei dem Komplexbildner handelt es sich zum Beispiel um Calciumsalze, Citrat oder EDTA. Besonders bevorzugt sind im Rahmen der vorliegenden Erfindung Calciumenthaltende Salze, wie z.B. Calciumchlorid. Die Konzentration des Calciumsalzes beträgt zwischen 0,005 und 0,05 g/l, bevorzugt zwischen 0,008 und 0,012 g/l und besonders bevorzugt 0,01 g/l.

Zur Einstellung der Osmolalität kann jedes geeignete Salz eingesetzt werden. Bevorzugt wird hierzu Natriumchlorid verwendet. Dabei liegt die Konzentration des Natriumchlorids im Bereich von 0,5 bis 2,5 g/l, bevorzugt zwischen 1,0 und 2,0 g/l und besonders bevorzugt zwischen 1,3 und 1,6 g/l.

Die Osmolalität der Flüssigformulierung beträgt 200 bis 400 mosmol/kg, bevorzugt 250 bis 300 mosmol/kg und am meisten bevorzugt 260 bis 290 mosmol/kg.

Eine bevorzugte Ausführungsform der Erfindung sieht eine EPO-Flüssigformulierung vor, die neben dem WirkstoffEPO die Aminosäuren Leucin, Isoleucin, Threonin, Glutaminsäure, Phenylalanin und Glycin sowie Calciumchlorid, Polysorbat, Phospatpuffer und Natriumchlorid enthält, wobei weitere Inhaltsstoffe nicht vorhanden sind.

Die Wirksamkeit des verwendeten Erythropoietins sollte nicht unter 100.000 IE/mg liegen (siehe auch Europäische Pharmacopoeia, 01/2002:1316 (4. Auflage). Bevorzugt hat das EPO eine Aktivität von mindestens 110.000 IE/mg, besonders bevorzugt von mindestens 120.000 IE/mg.

Die Konzentration an Erythropoietin richtet sich nach der jeweils erwünschten Wirkstoffkonzentration in der Fertigspritze. Typische EPO-Konzentrationen liegen zwischen 1500 IE/ml bis 40.000 IE/ml, siehe z.B. Handelsprodukte NeoRecormon^{®} und Erypo^{®} in ROTE LISTE 2004.

Die Komponenten der Formulierung können von üblichen Quellen bezogen werden, z.B. von der Firma Sigma oder der Firma Merck.

Die Herstellung der Formulierung kann nach im Stand der Technik üblichen Methoden erfolgen.

Die Erfindung betrifft auch ein Verfahren zur Herstellung der erfindungsgemäßen EPO-Flüssigformulierung. Dabei werden die oben genannten Bestandteile der Formulierung in einem wässrigen Puffer gelöst. Bei dem wässrigen Puffer handelt es sich, wie oben ausgeführt, vorzugsweise um einen Phosphatpuffer.

In einer bevorzugten Ausführungsform des erfindungsgemäßen Verfahrens zur Herstellung der Flüssigformulierung wird zunächst das Lösungsmittel, bevorzugt Wasser für Injektionszwecke, vorgelegt und anschließend Natriumdihydrogenphosphat-Dihydrat und Dinatriumhydrogenphosphat-Dihydrat als Puffer, Calciumchlorid, Natriumchlorid und die eingesetzten Aminosäuren, bei denen es sich bevorzugt um Glycin, Leucin, Isoleucin, Threonin, Glutaminsäure und Phenylalanin handelt, in dem Lösungsmittel gelöst. Nach vollständiger Auflösung der Bestandteile wird eine Lösung von Polysorbat (in dem jeweiligen Lösungsmittel) zugegeben. Schließlich wird im nächsten Schritt eine Lösung von Erythropoietin (in dem jeweiligen Lösungsmittel) zugegeben.

Während der Herstellung und am Ende der Herstellung wird der pH-Wert der Lösung bestimmt und falls erforderlich in den Bereich zwischen 7,0 und 7,4 korrigiert, unter Einsatz geeigneter Lösungen, insbesondere NaOH oder HCl.

Schließlich wird die fertige Flüssigformulierung in ein geeignetes Behältnis abgefüllt, in dem es bis zur Applikation gelagert wird. Bei dem Behältnis handelt es sich insbesondere um Fertigspritzen, Durchstichflaschen oder Ampullen.

Die folgenden Beispiele sollen die Erfindung erläutern, ohne sie einzuschränken.

### Beispiele

### 1. Erythropoietin-Flüssigformulierung

Herstellung einer Flüssigformulierung von Erythropoietin mit einer Wirkstoffstärke von 3.333 IE/ml.

| **Bestandteil** | **Gehalt pro Fertigspritze** |
|---|---|
| EPO | 3.333 IE/ml |
| Polysorbat 20 | 0,1 mg |
| Natriumdihydrogenphosphat-Dihydrat | 1,43 mg |
| Natriummonohydrogenphosphat-Dihydrat | 5,6 mg |
| CaCl₂ x 2 H₂O | 0,01 mg |
| Glycin | 7,5 mg |
| Leucin | 1,0 mg |
| Isoleucin | 1,0 mg |
| Threonin | 0,25 mg |
| Glutaminsäure | 0,25 mg |
| Phenylalanin | 0,5 mg |
| Natriumchlorid | 1,43 mg |
| Natriumhydroxid 0,1 n* | q.s. |
| Salzsäure 0,1 n* | q.s. |
| Wasser | ad 1,0 ml |
| Stickstoff** | q.s. |

| | |
|---|---|
| * Zur pH-Wert-Einstellung ** Zur Begasung der Lösung bei der Herstellung und Abfüllung | |

Sämtliche Inhaltsstoffe weisen eine Qualität gemäß Europäischer Pharmacopoeia (Ph. Eur.) auf.

### 2. Herstellung einer Flüssigformulierung für Erythropoietin mit einer Wirkstoffstärke von 10.000 IE/ml

Die Gehalte der einzelnen Bestandteile pro Fertigspritze entsprechen mit Ausnahme des Gehalts an Erythropoietin denen in Beispiel 1. Der Gehalt an Erythropoietin beträgt in diesem Fall 10.000 IE/ml.

Die Formulierungen werden in Glasspritzen abgefüllt und als Fertigspritzen gelagert.

### 3. Herstellung einer EPO-Flüssigformulierung mit einer Ansatzgröße von einem Liter

Bei einer exemplarischen Ansatzgröße von 11, einem beispielhaften Proteingehalt von 1.000 µg/ml und einer Aktivität von 130.000 IE/mg Protein ergibt sich die in nachfolgender Tabelle angegebene Zusammensetzung (angenommene Dichte des Wirkstoffbulks: 1,008 g/l).

Dabei wird eine Wirkstofflösung hergestellt, enthaltend Erythropoietin, Natriummonohydrogenphosphat-Dihydrat und Natriumdihydrogenphosphat-Dihydrat, Natriumchlorid und Wasser, deren Zusammensetzung in die Berechnung der einzuwiegenden Mengen der entsprechenden Bestandteile mit einfließt.

| **Ausgangsstoffe** | | |
|---|---|---|
| Ansatzmenge* | 11= 1.006,7 g | 1 1= 1.006,7 g |
| Wirkstoffstärke | 3.333 IE/ml | 10.000 IE/ml |
| Wirkstoffgehalt | 25,6 µg/ml | 76,9 µg/ml |
| Wirkstofflösung | 25,64 g | 76,97 g |
| Polysorbat 20 | 0,1 g | 0,1 g |
| Natriumdihydrogenphosphat-Dihydrat** | 1,43 g | 1,43 g |
| Natriummonohydrogenphosphat-Dihydrat** | 5,6 g | 5,6 g |
| CaCl₂ x 2 H₂O | 0,01g | 0,01g |
| Glycin | 7,5 g | 7,5 g |
| Leucin | 1,0 g | 1,0 g |
| Isoleucin | 1,0 g | 1,0 g |
| Threonin | 0,25 g | 0,25 g |
| Glutaminsäure | 0,25 g | 0,25 g |
| Phenylalanin | 0,5 g | 0,5 g |
| Natriumchlorid** | 1,43 g | 1,43 g |
| Natriumhydroxid 0,1 n*** | q.s. | q.s. |
| Salzsäure 0,1 n*** | q.s. | q.s. |
| Wasser** | 984,9 | 984,9 |
| Stickstoff | q.s. | q.s. |

| | | |
|---|---|---|
| * Die Ansatzgröße ergibt sich nach der eingesetzten Wirkstoffmenge und dem Proteingehalt, der mit Hilfe von RP-HPLC ermittelt wurde. ** Einzuwiegende Menge. In die Mengenberechnung fließt der Gehalt der Hilfsstoffe der Wirkstofflösung mit ein. *** Zur pH-Wert-Einstellung, verbrauchte Menge fließt in die Mengenberechnung mit ein. | | |

### 4. Verfahren zur Herstellung der EPO-Flüssigformulierung

80% des Wassers für Injektionszwecke werden in den Ansatzbehälter vorgelegt. Die Temperatur des Wassers wird kontrolliert, sie soll unter 25°C betragen. Anschließend erfolgt vorsichtig unter Rühren und Stickstoffschutz die Zugabe der entsprechenden eingewogenen Mengen laut oben angegebener Zusammensetzung: Natriumdihydrogenphosphat-Dihydrat, Dinatriumhydrogenphosphat-Dihydrat, Calciumchlorid, Glycin, Leucin, Isoleucin, Threonin, Glutaminsäure, Phenylalanin und Natriumchlorid. Die Lösung wird bis zur vollständigen Auflösung aller Bestandteile zu einer homogenen Lösung, mindestens aber 15 Minuten gerührt. Anschließend wird das Polysorbat 20 in Form einer separat hergestellten Lösung in Wasser für Injektionszwecke in den Ansatzbehälter gegeben. Die Lösung wird mindestens 15 Minuten gerührt.

Zur pH-Wert-Einstellung werden im Ansatzbehälter Temperatur und pH-Wert des Ansatzes überprüft. Die Temperatur soll zwischen 18 und 25°C liegen. Der pH-Wert soll in einem Bereich von 7,0 - 7,4 liegen. Liegt der pH-Wert über 7,4, wird er mit 0,1 n Salzsäure eingestellt. Liegt der pH-Wert unter pH 7,0, wird die Korrektur mit 0,1 n Natriumhydroxid-Lösung vorgenommen. Die Lösung im Ansatzbehälter wird 10 Minuten gerührt.

Nach Einstellung des pH-Wertes wird die entsprechende Menge des Erythropoietin-Proteins in den Ansatzbehälter gegeben.

Nochmals werden die Temperatur (Soll zwischen 18 und 25°C) und der pH-Wert des Ansatzes überprüft. Liegt der pH-Wert über 7,4 bzw. unter 7,0 wird mit 0,1 n Salzsäure resp. 0,1 n Natriumhydroxid-Lösung korrigiert.

Mit Wasser für Injektionszwecke wird auf das berechnete Endgewicht der Lösung im Ansatzbehälter aufgefüllt und anschließend mindestens 15 Minuten gerührt. Gegebenenfalls muss der pH-Wert noch einmal wie oben angegeben in den Bereich zwischen 7,0 und 7,4 korrigiert werden. Die fertige EPO-Flüssigformulierung wird in Fertigspritzen abgefüllt, die nach dem Befüllen mit Kolbenstopfen verschlossen werden.

### 5. Langzeitstabilität der erfindungsgemäßen EPO-Formulierungen

Die Langzeitstabilität der erfindungsgemäßen EPO-Formulierungen wurde im Vergleich zu dem BRP-Standard als Referenz überprüft. Der Test beruht darauf, dass bei der Lagerung von EPO-Lösungen Abbau- und Nebenreaktionen auftreten, die u.a. zur Oxidation von Methionin- und Cystein-Seitenketten führen können. Insbesondere Met54 von EPO wird häufig unter Bildung des entsprechenden Sulfoxids oxidiert. Dieses Methioninsulfoxid kann nach proteolytischem Verdau des EPO mit Trypsin durch eine rp-HPLC-Analyse detektiert werden. Der Anteil an Methionin-oxidierten Spezies ist dabei ein Indikator für die Lagerstabilität, d.h. je höher der Anteil, desto geringer die Lagerstabilität. Die Bestimmung des Anteils an Methionin-oxidierten Spezies in den erfindungsgemäßen Formulierungen bei verschiedenen Lagertemperaturen (2-8°C, 25°C und 40°C) und verschiedenen Lagerzeiträumen (6 Wochen, 12 Wochen und 6 Monate) im Vergleich zu Formulierungen des Standes der Technik zeigte, dass die erfindungsgemäßen Formulierungen eine Stabilität aufweisen, die mit der der Flüssigformulierungen des Standes der Technik vergleichbar ist.

## Patentansprüche

1. Lagerstabile Erythropoietin-Flüssigformulierung, die
(i) mindestens vier Aminosäuren ausgewählt aus der Gruppe bestehend aus Leucin, Isoleucin, Threonin, Glutaminsäure, Asparaginsäure und Phenylalanin enthält,
(ii) frei von Konservierungsmitteln, Harnstoff und HSA ist, und
(iii) nicht aus einem Lyophilisat rekonstitutiert wurde.

2. Formulierung nach Anspruch 1, wobei des weiteren Glycin enthalten ist.

3. Formulierung nach Anspruch 1 oder 2, weiter umfassend ein nichtionogenes Detergenz.

4. Formulierung nach Anspruch 3, wobei es sich bei dem Detergenz um einen Polyoxyethylensorbitanalkylester handelt.

5. Formulierung nach Anspruch 4, wobei es sich bei dem Polyoxyethylensorbitanalkylester um Polysorbat 20 oder Polysorbat 80 handelt.

6. Formulierung nach einem der Ansprüche 1 bis 5, wobei die Osmolalität 250 mosmol/kg bis 300 mosmol/kg beträgt.

7. Formulierung nach Anspruch 6, wobei die Osmolalität mit Natriumchlorid eingestellt ist.

8. Formulierung nach einem der Ansprüche 1 bis 7, weiter enthaltend einen physiologisch verträglichen Puffer.

9. Formulierung nach Anspruch 8, wobei der Puffer ein Phosphatpuffer ist.

10. Formulierung nach einem der Ansprüche 1 bis 9, wobei der pH-Wert zwischen 7,0 und 7,4 liegt.

11. Formulierung nach einem der Ansprüche 1 bis 10, weiter enthaltend Calciumionen.

12. Formulierung nach Anspruch 11, wobei die Calciumionen aus Calciumchlorid stammen.

13. Formulierung nach einem der Ansprüche 1 bis 12, wobei die Konzentration von Isoleucin und Leucin, soweit enthalten, jeweils 0,25 bis 1,5 g/l beträgt.

14. Formulierung nach einem der Ansprüche 1 bis 13, wobei die Konzentration von Glutaminsäure und Asparaginsäure, soweit enthalten, 0,1 bis 0,5 g/l beträgt.

15. Formulierung nach einem der Ansprüche 1 bis 14, wobei die Konzentration von Threonin, soweit enthalten, 0,1 bis 0,5 g/l beträgt.

16. Formulierung nach einem der Ansprüche 1 bis 15, wobei die Konzentration von Phenylalanin, soweit enthalten, 0,2 bis 1,0 g/l beträgt.

17. Formulierung nach einem der Ansprüche 2 bis 16, wobei die Konzentration von Glycin, soweit enthalten, 2,0 bis 10,0 g/l beträgt.

18. Verfahren zur Herstellung einer lagerstabilen Erythopoietin-Flüssigformulierung umfassend: Lösen der Bestandteile Erythropoietin, der Aminosäuren Leucin, Isoleucin, Threonin, Glutaminsäure, Phenylalanin und Glycin sowie Calciumchlorid, Polyoxyethylensorbitanalkylester, Natriumchlorid in einem wässrigen Puffer, wobei die Formulierung in keiner Phase des Verfahrens lyophilisiert wird und die Formulierung frei von Harnstoff ist.

19. Verfahren nach Anspruch 18, wobei die Bestandteile in der folgenden Reihenfolge gelöst werden:
a) Calciumchlorid,
b) Natriumchlorid,
c) Leucin, Isoleucin, Threonin, Glutaminsäure, Phenylalanin und Glycin,
d) Polyoxyethylensorbitanalkylester und
e) Erythropoietin.

## Claims

1. Stable liquid erythropoietin preparation,
(i) containing at least four amino acids selected from the group consisting of leucine, isoleucine, threonine, glutamic acid, aspartic acid and phenyl alanine,
(ii) being free of preservatives, urea and HSA, and
(iii) not being reconstituted from lyophilisate.

2. Preparation according to claim 1, further containing glycine.

3. Preparation according to claim 1 or 2, further comprising non-ionogenic detergent.

4. Preparation according to claim 3, wherein the detergent is a polyoxy ethylene sorbitan alkyl ester.

5. Preparation according to claim 4, wherein the polyoxy ethylene sorbitan alkyl ester is Polysorbate 20 or Polysorbate 80.

6. Preparation according to any of claims 1 to 5, wherein the osmolality is in the range of 250 mosmol/kg to 300 mosmol/kg.

7. Preparation according to claim 6, wherein the osmolality is adjusted with sodium chloride.

8. Preparation according to any of claims 1 to 7, further comprising a physiologically acceptable buffer.

9. Preparation according to claim 8, wherein the buffer is a phosphate buffer.

10. Preparation according to any of claims 1 to 9, wherein the pH is in the range of 7.0 and 7.4.

11. Preparation according to any of claims 1 to 10, further comprising calcium ions.

12. Preparation according to claim 11, wherein the calcium ions are from calcium chloride.

13. Preparation according to any of claims 1 to 12, wherein the concentration of isoleucine and leucine, if contained, is each in the range of 0.25 to 1.5 g/l.

14. Preparation according to any of claims 1 to 13, wherein the concentration of glutamic acid and aspartic acid, if contained, is in the range of 0.1 to 0.5 g/l.

15. Preparation according to any of claims 1 to 14, wherein the concentration of threonine, if contained, is in the range of 0.1 to 0.5 g/l.

16. Preparation according to any of claims 1 to 15, wherein the concentration of phenyl alanine, if contained, is in the range of 0.2 to 1.0 g/l.

17. Preparation according to any of claims 2 to 16, wherein the concentration of glycine, if contained, is in the range of 2.0 to 10.0 g/l.

18. Process for preparing a storage stable erythropoietin liquid formulation comprising dissolving erythropoietin, the amino acids leucine, isoleucine, threonine, glutamic acid, phenylalanine and glycine as well as calcium chloride, polyoxyethylene sorbitan alkylester, sodium chloride in an aqueous buffer, wherein the formulation is not lyophilised in any stage of the process and the formulation is free of urea.

19. Process according to claim 18, wherein the components are dissolved in the following order:
a) calcium chloride,
b) sodium chloride,
c) leucine, isoleucine, threonine, glutamic acid, phenyl alanine and glycine,
d) polyoxy ethylene sorbitan alkyl ester and
e) erythropoietin.

## Revendications

1. Formule liquide d'érythropoïétine stable au stockage, qui
(i) contient au moins quatre acides aminés choisis dans le groupe constitué de la leucine, l'isoleucine, la thréonine, l'acide glutamique, l'acide asparagique et la phénylalanine,
(ii) ne contient ni conservateurs, ni urée, ni albumine sérique humaine et
(iii) n'a pas été reconstituée à partir d'un lyophilisat.

2. Formule selon la revendication 1, qui contient, en outre, de la glycine.

3. Formule selon la revendication 1 ou 2, comprenant, en outre, un détergent non ionogène.

4. Formule selon la revendication 3, dans laquelle le détergent est un ester alkylique de type polyoxyéthylène sorbitane.

5. Formule selon la revendication 4, dans laquelle l'ester alkylique de type polyoxyéthylène sorbitane est du polysorbate 20 ou du polysorbate 80.

6. Formule selon l'une quelconque des revendications 1 à 5, dans laquelle l'osmolalité est comprise entre 250 mosmole/kg et 300 mosmole/kg.

7. Formule selon la revendication 6, dans laquelle l'osmolalité est ajustée avec du chlorure de sodium.

8. Formule selon l'une quelconque des revendications 1 à 7, qui contient, en outre, un tampon physiologiquement compatible.

9. Formule selon la revendication 8, dans laquelle le tampon est un tampon phosphate.

10. Formule selon l'une quelconque des revendications 1 à 9, dans laquelle le pH est compris entre 7,0 et 7,4.

11. Formule selon l'une quelconque des revendications 1 à 10, qui contient, en outre, des ions calcium.

12. Formule selon la revendication 11, dans laquelle les ions calcium proviennent du chlorure de calcium.

13. Formule selon l'une quelconque des revendications 1 à 12, dans laquelle la concentration en isoleucine et en leucine, dans la mesure où elles figurent, est pour chacune comprise entre 0,25 et 1,5 g/l.

14. Formule selon l'une quelconque des revendications 1 à 13, dans laquelle la concentration en acide glutamique et en acide asparagique isoleucine, dans la mesure où ils figurent, est pour chacun comprise entre 0,1 et 0,5 g/l.

15. Formule selon l'une quelconque des revendications 1 à 14, dans laquelle la concentration en thréonine, dans la mesure où elle figure, est comprise entre 0,1 et 0,5 g/l.

16. Formule selon l'une quelconque des revendications 1 à 15, dans laquelle la concentration en phénylalanine, dans la mesure où elle figure, est comprise entre 0,2 et 1,0 g/l.

17. Formule selon l'une quelconque des revendications 2 à 16, dans laquelle la concentration en glycine, dans la mesure où elle figure, est comprise entre 2,0 et 10,0 g/l.

18. Procédé de fabrication d'une formule liquide d'érythropoïétine stable au stockage, qui comprend : la solution des composantes érythropoïétine, des acides aminés leucine, isoleucine, thréonine, acide glutamique, phénylalanine et glycine ainsi que chlorure de calcium, ester alkylique de type polyoxyéthylène sorbitane, chlorure de sodium dans un tampon aqueux, la formule n'étant lyophilisée dans aucune phase et la formule ne contenant pas d'urée.

19. Procédé selon la revendication 18, dans laquelle les composantes sont dissoutes dans l'ordre suivant :
a) chlorure de calcium,
b) chlorure de sodium,
c) leucine, isoleucine, thréonine, acide glutamique, phénylalanine et glycine,
d) ester alkylique de type polyoxyéthylène sorbitane et
e) érythropoïétine.
